# EUROPÄISCHE PATENTSCHRIFT

(11) **EP 3 115 464 B1**
(45) Veröffentlichungstag und Bekanntmachung des Hinweises auf die Patenterteilung: **31.05.2017**
(21) Anmeldenummer: 15175817.4
(22) Anmeldetag: 08.07.2015
(51) Int. Cl.: C12Q 1/68

(54) **VERFAHREN ZUR QUALITÄTSSICHERUNG BEI DER TRINKWASSER- UND/ODER GRUNDWASSERVERSORGUNG**
METHOD FOR QUALITY ASSURANCE IN THE SUPPLY OF DRINKING WATER AND/OR GROUND WATER
PROCEDE D'ASSURANCE QUALITE POUR L'EAU POTABLE ET/OU L'APPROVISIONNEMENT EN EAU SOUTERRAINE

(43) Veröffentlichungstag der Anmeldung: 11.01.2017
(73) Patentinhaber: Institut für Grundwasserökologie IGÖ GmbH, 76829 Landau (DE)
(72) Erfinder: HAHN, Hans Jürgen, 76829 Landau (DE)
(74) Vertreter: Patentanwälte Dr. Keller, Schwertfeger

(56) Entgegenhaltungen:
- WO-A1-2007/115590
- CN-B- 102 703 586
- KR-B1- 100 781 206
- M Konec ET AL: "Microsatellite loci for the study of cave populations of Asellus aquaticus" In: "Molecular Ecology Resources", 31. März 2011 (2011-03-31), In: Austin J.D. et al.: Permanent genetic resources added to Molecular Ecology Resources Database, XP055216555, Bd. 11, Seiten 757-758, * Zusammenfassung * * Tabellen 1-2 * -& JAMES D. AUSTIN ET AL: "Permanent Genetic Resources added to Molecular Ecology Resources Database 1 February 2011-31 March 2011", MOLECULAR ECOLOGY RESOURCES, Bd. 11, Nr. 4, 31. Juli 2011 (2011-07-31), Seiten 757-758, XP055216553, ISSN: 1755-098X, DOI: 10.1111/j.1755-0998.2011.03028.x
- M. KONEC ET AL: "Parallels between two geographically and ecologically disparate cave invasions by the same species, Asellus aquaticus (Isopoda, Crustacea)", JOURNAL OF EVOLUTIONARY BIOLOGY, Bd. 28, Nr. 4, 10. April 2015 (2015-04-10) , Seiten 864-875, XP055216532, ISSN: 1010-061X, DOI: 10.1111/jeb.12610 -& "Appendix S2: Localities and GenBank accession numbers Locality, nearby city, country code Site code on Figure 1 COI haplotypes 28S rDNA sequences Sample size Used in Central Europe network and tree 1 Ålborg", , 10. März 2015 (2015-03-10), XP055216542, Gefunden im Internet: URL:http://onlinelibrary.wiley.com/store/1 0.1111/jeb.12610/asset/supinfo/jeb12610-su p-0002-AppendixS2.xlsx?v=1&s=3d7aa8026e417 2a3a09705d3574f296297f4df47 [gefunden am 2015-09-28]
- TetraTech Inc. und Herrera Environmental Consultants (Hrsg.): "Using Microbial Source Tracking to Support TMDL Development and Implementation", , 1. April 2011 (2011-04-01), XP055217331, Gefunden im Internet: URL:http://www3.epa.gov/region10/pdf/tmdl/ mst_for_tmdls_guide_04_22_11.pdf [gefunden am 2015-09-30]
- ETIENNE KORNOBIS ET AL: "Discordance in Variation of the ITS Region and the Mitochondrial COI Gene in the Subterranean Amphipod", JOURNAL OF MOLECULAR EVOLUTION, SPRINGER-VERLAG, NE, Bd. 73, Nr. 1 - 2, 4. August 2011 (2011-08-04), Seiten 34-44, XP019983618, ISSN: 1432-1432, DOI: 10.1007/S00239-011-9455-2
- CALDWELL JANE M ET AL: "Mitochondrial multiplex real-time PCR as a source tracking method in fecal-contaminated effluents", ENVIRONMENTAL SCIENCE & TECHNOLOGY, AMERICAN CHEMICAL SOCIETY, US, Bd. 41, Nr. 9, 1. Mai 2007 (2007-05-01), Seiten 3277-3283, XP009120244, ISSN: 0013-936X, DOI: 10.1021/ES062912S

### SEQUENCE LISTING

<110> Institut für Grundwasserökologie IGÖ GmbH
<120> Verfahren zur Qualitätssicherung bei der Trinkwasser- und/oder Grundwasserversorgung
<130> StygoTracing
<160> 10
<170> PatentIn version 3.5
<210> 1
   <211> 510
   <212> DNA
   <213> Asellus aquaticus
<400> 1
   aaaataaaca aagaagattt ttaaatagca gaaaacttat tataaatcta attctattgc 60
   taaaattagt taaatgcgaa caattatctc gtataaggag ctcccagctg cctattaatt 120
   gaaacgtgat taaaatatta ttggaacaca aataagttat tgaaacgtct ttagaggaaa 180
   ctatttggct tcttaactcc agtctaattg cagactcttg acgcgaattg gcagcctcga 240
   taaaacactc atcgggagaa acgcccagat caaactctcg ccttggaacc taaccgttaa 300
   agctaattac acacacacac acacacacac acaattattg tatccaactt atgttgtaac 360
   gtacaggtaa gtatagtggc ggctcgtgac ataatacttt ggggggtgct gtatcaaatg 420
   tcaaaacaca gctatatgta aagytatact gcgtgcctac ctgcatgtgt gtgtgcgttt 480
   gtttagacac acttgcgagg tatttttaag 510
<210> 2
   <211> 505
   <212> DNA
   <213> Asellus aquaticus
<400> 2
   ctctatcgct gcacctaccc accctctctc tctctctctc tctctctctc acccactccc        60
   ttaataaagg accacggagg aactgcggac ggaccatgtt gtattgttta aatatattat       120
   aaacggcgtg acggcatcat tgtttttata atctatcagc tgatgccagg cgtctccgat       180
   gggcagccac cgctatttat cctccatttc aaaagcakta atatttaaat aaatttttcc       240
   gactcgattt aaataaacat tgaaacattc gcgttttaaa attaaaattg gcgtcgttaa       300
   aatttkactt aataaaagaa tccttcttgt ttatcttgat ttacgaaagg agttaatcac       360
   gatcgtccta tgggagcaat cctatcttga tcaaccattt agttttagag tgaactaaac       420
   tagccaatga ttagggcgtg tggccaaatg gatgggttca aggatgttgt cagtgtttat       480
   gtcggttcta tcgcccttac atctc                                             505
<210> 3
   <211> 348
   <212> DNA
   <213> Asellus aquaticus
<400> 3
   gtagcaattt gtcggtactt ggaaaaaatt gtaattgcat atacaaatat ttgtcttttt        60
   ggtaaactag agaatattaa aagggaatgt tggtacatac aacacgtact ttaagcatta       120
   ataaattata gttggaaaat tacatacaaa ctaggaagcc agaggttatc aacttatcaa       180
   gccaacccta ctcttgagaa ttataacttc ctagggtgtt taaaacttca tgacacacac       240
   acacacacac acacacgcac aaacacaagc tgccgctggc tattttacaa ccacggtaca       300
   cttggtatgg tttagatata attcttttat actttaatga aaaaagcg                    348
<210> 4
   <211> 345
   <212> DNA
   <213> Asellus aquaticus
<400> 4
   ttctacgtac ggtaaacact aactattttt gcgggcatag tacgtatgta cactgtatat        60
   acgttgttya atatcaaatc ttctgaaatt tttaaattaa tgtttgttgc cttcaaaatg       120
   agattggtta agataattta aataacaatt tctgtccttg gaagaaagag agagagagag       180
   agagagagag aaagagatga ttagattcct cccacgaaca ttcaatttgt tcgtcgagta       240
   tttgtctttc taattacgct gtcgatgaat gaaagtaaaa tattaactaa agggtgtcga       300
   cgtgccctta aaagtcaact ttacaacatt tcaaaatata tttat                       345
<210> 5
   <211> 259
   <212> DNA
   <213> Asellus aquaticus
<400> 5
   catcgatcca tacagcaaca agtgaagagc tggcacatat ttgaattttt ccctcacttc 60
   agtgtctctt ctctgtgtcc ttcctctccc gggtcctagg tctcccttcc acatctctct 120
   cgctctctct cgctctctca ctctctctct ctctctctct ctctcgaaac ttatagatct 180
   ctctaacaat tgacgtcata agaatttcac cctatggctc aaaatccttt tcatttttat 240
   gcttatttgg tttagttat 259
<210> 6
   <211> 258
   <212> DNA
   <213> Asellus aquaticus
<400> 6
   agccctttta ctgtcaaagc ttcgtcaggg taaacaagat gctcaaggca aaaacaataa 60
   actgaatcca gatcaagttc caggtgacta tactttttta caaagagaga cagttgaaaa 120
   gagagagaga gagagagaga gagagagaga gagaggagga ggaagaagta aagagaaata 180
   attcctttga taatattttc caatcaaggc ggcgcttgga gtatcttgtt cctcattatt 240
   gttgttgtcc ctcagtca 258
<210> 7
   <211> 356
   <212> DNA
   <213> Asellus aquaticus
<400> 7
   gtgaggcagt gaaataaaag aataaaattt aagaaaaaac attaaattga ggcagagaac 60
   acgattaagg agggggatac agagagaaat aragagggct cttggaatag ggagaaagaa 120
   ggagatagaa aaacggagtg atggaaagat agtattgaat aagagagaga gagagagaga 180
   gagagggagc gaacggggag agcggacgag agattaatat tagcgctgaa tattttgatt 240
   gagcggggga ctcaaatgag gcgataggga gtttggcaaa aataatctgc cattacagta 300
   tttcatttgg gaataatatt aatacattaa gaaataatga cgtctgcgtt ctcctt 356
<210> 8
   <211> 354
   <212> DNA
   <213> Asellus aquaticus
<400> 8
   attgttatat catcttgatt tggcatgaaa ttattttctt ctttctctca tggattcagt 60
   tgctttattc tcttcatttt cgctttttat ttgctgacaa aatacgaagg attgagtgtg 120
   aaagagagag aacagagaga gagagagaga gagagagaga gcaactgatg gacgtcgtct 180
   tcgggagtga aacgaatttg gaggtttaat tttgagacaa attaaaaaca ataagagaat 240
   cagagagtga gaaggaaaga gaagaatagg agagaggrag agagagagag aaggaaagag 300
   aagaatagga gagagggaga gagagagagt tacaaaaaat aaagcttaac aaat 354
<210> 9
   <211> 455
   <212> DNA
   <213> Asellus aquaticus
<400> 9
   gtcttaatag agagagagag ggactgagag agagagaggg attgagagag agagagagat 60
   tgagagagag agagagattt gaagaagaga gaacgacctt taattttagc acacccttcc 120
   ggtttctcac atacgtcttt cggctcattt tatctggtgc ttgggtttga tcttcatacg 180
   tcgttagatc ggaagaccct cggcctctct ctctctctct ctctctctct ctctctctct 240
   ctctctctct ctctcgacct gctcatttat ttctctctaa ttggattgat ctaaaacgag 300
   gaaacgcctt atagaaagaa attaaaatct gtatatagag ggaggagtcc gtgtacgtgc 360
   gtgaacgtat tccgagaatt aaattccgta ctttttaatt acccgtaact tctataatac 420
   gataatgctt ttcttattta taaaccattc ctgta 455
<210> 10
   <211> 392
   <212> DNA
   <213> Asellus aquaticus
<400> 10
   cacgaaaatt agctacaatt acaaattcat attgacaagg ttattgggta gaaagttaat 60
   ttgaatctac acagaatttg gattattttg gaagacaaga aagagaaaga gagagagaga 120
   gagagagaga gagagtgttt ctcacgcgtt aattaatcca caagctcgat gaatgaattt 180
   taaatagact gataacgtac aaaatttaaa ttaacattgt attagttccg ttttatttct 240
   tcaataattt aagcgatttt tttttgtata ttaggtcacc cgactacatt aaaaacgata 300
   caaaatatct tacttgcatc agtttctctc agtttctttc ctttcccaag tccaatctca 360
   aatctcgcac atctttcctc tcttctcttc at 392

## Beschreibung

Die vorliegende Erfindung betrifft ein Verfahren zur Qualitätssicherung bei der Trinkwasser- und/oder Grundwasserversorgung in Trinkwasser- und/oder Grundwassersystemen durch eine populationsgenetische Analyse von Mikrosatelliten in der DNA von wenigstens einem Tracerorganismus.

Die Erfindung betrifft ferner die für eine Mikrosatellitenanalyse entwickelten Nukleinsäureprimer für die Durchführung einer Polymerasenketten-Reaktion (PCR) sowie deren Verwendung zur Analyse und/oder Charakterisierung von Kleinlebewesen in einem Trinkwasser- und/oder Grundwassersystem.

Ein Großteil des weltweit verfügbaren Trinkwassers basiert auf Grund- und Quellwasser. Die Qualität des Trinkwassers ist in hohem Maße von der Grundwasserqualität abhängig. Für die Reinigung von Grundwasser sind unter anderem natürlich vorkommende Mikroorganismen verantwortlich.

Der Eintrag von Fremdorganismen, insbesondere solchen, die aus Oberflächengewässern stammen, stellt ein häufiges Problem bei gängigen Trinkwasser- oder Grundwassersystemen dar. Viele dieser Organismen neigen zu Massenpopulationen, was zu hygienischen Problemen führen kann. Zu solchen Kleintieren (Invertebraten) gehören beispielsweise die Wasserasseln (*Asellus aquaticus*). Bei Trinkwassersystemen stellt das Versorgungsnetz einen Lebensraum für diese Kleintiere dar, die sich von den darin enthaltenen Biofilmen und organischen Ablagerungen ernähren.

Während chemische Kontaminanten im Trinkwasser leicht und reproduzierbar nachgewiesen werden können, ist der gesicherte quantitative Nachweis von Invertebraten im Trinkwasser weitaus schwieriger. Im Allgemeinen bedeutet das Auftreten von tierischen Organismen für den Menschen keine konkrete Gesundheitsgefahr, allerdings treten in solchen Trinkwassersystemen auch durch Asselkot oder tote Asseln bedingte Sekundärverkeimungen auf, was zu einer Verschlechterung der Wasserqualität führt.

Die Verteilung von Wasserasseln in einem Trinkwasserverteilungssystem wurde bereits untersucht. Neben der Wasserassel (*Asellus aquaticus*) wurden in den untersuchten Proben zahlreiche weitere Taxa festgestellt (Gunkel G. et al., Vorkommen und Bedeutung von Kleintieren im Trinkwasserverteilungssystemen, Fachbericht Wasserversorgung, S. 716-724, 12/2010).

Für die Bekämpfung von Wasserasseln wurden verschiedene physikalische Verfahren (beispielsweise UV, Ultraschall und Temperatur, CO₂, Luft-Wasser-Spülungen) oder chemische Bekämpfungsverfahren mit Chlor eingesetzt, was jedoch zumeist wenig erfolgreich war. Der Einsatz von Bioziden ist in vielen Ländern nicht zulässig.

Über die Eintragspfade oder das Ausbreitungsverhalten solcher Kleintiere in Trinkwasser- und/oder Grundwassersystemen ist bislang wenig bekannt. Zur Untersuchung der Fließvorgänge in Grundwasser oder anderem Gewässer werden häufig Markierungsmittel, sogenannte Tracer, eingesetzt. Wichtige Voraussetzungen für Tracer sind neben einer guten Wasserlöslichkeit eine geringe Sorptionsneigung und chemische Stabilität. Meist werden hierzu Fluoreszenzfarbstoffe oder Salze verwendet. Je nach Fragestellung können jedoch auch radioaktive Tracer, Sporen oder Bakterien eingesetzt werden (Wernli, H.R. (2011): Einführung in die Tracerhydrologie (Hydrologisches Praktikum): Geographisches Institut der Universität Bern, Bern).

Daneben kommen zur Identifizierung von pathogenen Mikroorganismen bzw. Fäkalkeimen genetische Tracer in Gewässern oder in Trinkwasser zum Einsatz. Mit Hilfe eines mikrobiellen Verfahrens ("microbiosource trackings") können fäkale oder pathogene Kontaminationen im Wasser anhand spezifischer Mikroorganismen bis zu ihrem Ursprung zurückverfolgt werden.

Solche Verfahren basieren beispielsweise auf Microbial Source Tracking (MST), um die maximale tägliche Gesamtbelastung (Total Maximum Daily Loads; TMDL) zu bestimmen (Tetra Tech, Inc. und Herrera Environmental Consultants: "Using Microbial Source Tracking to Support TMDL Development and Implementation", 1. April 2011).

Daneben sind mikrosatellitenbasierte Populationsanalysen bei Tieren bekannt (Thielsch, A.; Völker, F.; Kraus, R.H.S.; Schwenk, K. (2012): Discrimination of hybrid classes using cross-species amplification of microsatellite loci: methodological challenges and solutions in Daphnia. - Molecular Ecology Resources); (Dlouhá, S.; Thielsch, A.; Kraus, R.H.S.; Seda, J.; Schwenk, K.; Petrusek, A. (2010): Identifying hybridizing taxa within the Daphnia longispina complex: a comparison of genetic methods and phenotypic approaches. - Hydrobiologia 643, 107-122).

So lassen sich beispielsweise auch Wanderbewegungen von Wildtieren analysieren (Kolodziej et al., 2013).

In technischen Anlagen oder zur Qualitätssicherung bei Grund- und Trinkwassersystemen wurden mikrosatellitenbasierte Populationsanalysen bislang nicht eingesetzt.

Bei Mikrosatelliten handelt es sich um kurze DNA-Bereiche, in denen ein Muster (Motiv) aus wenigen Basen mehrfach wiederholt wird. Mikrosatelliten können eine unterschiedliche Länge aufweisen, wobei bereits Wiederholungen von 1 bis 6 Basenpaaren eines Sequenzmotivs als Mikrosatelliten bezeichnet werden.

Änderungen in der Länge der Mikrosatelliten nehmen mit steigender Motiv-Wiederholung zu. Mit zunehmender Länge eines Mikrosatelliten nimmt auch die Chance von Punktmutationen innerhalb des Mikrosatelliten zu, wodurch dessen Länge stabilisiert wird.

So wurden Mikrosatelliten-Analysen bei der Wasserassel *Asellus aquaticus* durchgeführt, bei der dreizehn neue Mikrosatellitenloci identifiziert werden konnten (M. Konec, P. Trontelj, "Microsatellite loci for the study of cave populations of Asellus aquaticus", University of Ljubljana, Biotechnical Faculty, Department of Biology, Vecna pot 111, SI-1000 Ljubljana, Slovenia, Molecular Ecology Resources (2011; James D. Austin et al.: Permanent genetic resources added to Molecular Ecology Resources Database, 1 February 2011 - 31 March 2011, Molecular Ecology Resources (2011) 11, 757-758)).

Bei *Asellus aquaticus* wurde ferner der Grad der phänotypischen Abweichung bei zwei unabhängigen Höhlensystemen in Rumänien bzw. Slowenien untersucht (M. Konec, et al: "Parallels between two geografically and ecologically disparate cave invasions by the same species, Asellus aquaticus (Isopoda, Crustacea)", Journal of Evolutionary Biology, Bd. 28, (2015) 864-875). Dabei wurde festgestellt, dass die Gesamtmorphologie sich an den beiden Untersuchungsstellen signifikant verändert hat. Viele der beobachteten morphologischen Veränderungen sind nicht nur auf genetische Variationen zurückzuführen, sondern auch auf Umgebungsfaktoren, die auf die Entwicklung der Organismen Einfluss nehmen.

Vor dem Hintergrund der sich wiederholenden Problematik der Kontamination von Trinkwasser- oder Grundwassersystemen mit Kleinlebewesen bzw. mit Oberflächenwasser ist es Aufgabe der vorliegenden Erfindung, ein alternatives Verfahren zur Qualitätssicherung bei der Trinkwasser- und/oder Grundwasserversorgung in solchen Trinkwasser- und/oder Grundwassersystemen bereitzustellen.

Diese Aufgabe wird gelöst durch ein Verfahren mit den Merkmalen des Anspruchs 1.

Das erfindungsgemäße Verfahren basiert auf einer populationsgenetischen Analyse von Mikrosatelliten in der DNA von ausgewählten Tracerorganismen. Als Tracerorganismus wird ein Organismus bezeichnet, der als Lebewesen in einem Trinkwasser- oder Grundwassersystem vorkommen kann. Hierbei handelt es sich üblicherweise um stygobionte oder stygophile Invertebraten. Vorzugsweise wird eine Analyse von mehr als einem Tracerorganismus, bevorzugt mit mehreren unterschiedlichen Populationen von Tracerorganismen durchgeführt.

Vorzugsweise werden mehrere Mikrosatelliten an unterschiedlichen Loci in der DNA von wenigstens einem Tracerorganismus analysiert. Je mehr Bereiche analysiert werden, umso genauer kann eine Kartierung und somit Feststellung der Eintrags- bzw. Austragspfade der Tracerorganismen in das Trinkwassersystem oder Grundwassersystem erfolgen. Erfindungsgemäß werden mindestens 5, vorzugsweise 10 Mikrosatelliten-Loci eines Tracerorganismus analysiert. Anhand der so gewonnenen Ergebnisse können der Verwandtschaftsgrad und die genetische Herkunft ermittelt werden.

Erfindungsgemäß wird hierzu in einem ersten Verfahrensschritt wenigstens ein für das jeweilige Trinkwasser- und/oder Grundwassersystem geeigneter Tracerorganismus durch eine taxonomische Untersuchung und/oder genetische Charakterisierung ausgewählt. Das Auswählen der Tracerorganismen durch taxonomische Untersuchung kann beispielsweise anhand morphologischer Merkmale erfolgen. Daneben ist auch genetische Charakterisierung umsetzbar, sofern sich Tracerorganismen taxonomisch nicht voneinander unterscheiden lassen. Dies ist im Wesentlichen bei kryptischen Arten der Fall. Die genetische Charakterisierung der potentiellen Tracerorganismen ermöglicht die Identifizierung solcher kryptischen Arten. Bei der vorliegenden Erfindung wurden beispielsweise13 natürlich vorkommende *Asellus* aquaticus-Populationen mit insgesamt 172 Individuen in ganz Europa gesammelt, deren DNA-Sequenzen ermittelt und anschließend ausgewertet.

Im Grundwasser ist der Anteil von kryptischen Arten sehr hoch. Um festzustellen, ob eine kryptische Art vorliegt, wird eine phylogenetische Auswertung durchgeführt. Als Tracerorganismus eignen sich solche Arten, die entweder nicht kryptisch sind oder ein spezifisches regionales Verbreitungsmuster aufweisen. Vorzugsweise wird das Verfahren mit nicht kryptischen Arten durchgeführt oder mit kryptischen Arten, die regionale Verbreitungsmuster aufweisen.

Um die Herkunft, Einbring- oder Ausbringpfade in einem Trinkwasser- oder Grundwassersystem zu identifizieren, werden erfindungsgemäß an repräsentativen Stellten der Anlagen bzw. des Untersuchungsgebietes (Rohrnetz oder Freiland) Proben entnommen. Die Probenentnahme kann beispielsweise über entsprechende Auffangvorrichtungen im Durchlaufverfahren erfolgen. Dabei erfolgt eine erste morphologische Klassifizierung anhand der für die Gattung oder Art bekannten Merkmale. Arten, die im untersuchten Trinkwasser- oder Grundwassersystem besonders häufig auftreten und auch an unterschiedlichen Stellen anzutreffen sind, sind potentiell als Tracerorganismus geeignet. Vorzugsweise handelt es sich hierbei um stygobionte oder stygophile Organismen, d.h. obligatorische bzw. fakultative Grundwasserbewohner.

In einem weiteren Verfahrensschritt werden polymorphe Nukleinsäureprimer für eine Mikrosatellitenanalyse bereitgestellt. Sofern solche Primer für den jeweiligen Tracerorganismus und die zu untersuchenden Mikrosatelliten-Loci nicht verfügbar sind, können sie anhand üblicher Methoden hergestellt werden. Die Primer werden vorzugsweise so entworfen, dass sie die Mikrosatelliten flankieren und möglichst voneinander unterscheidbare DNA-Fragmente produzieren. Hierzu wird Probematerial von unterschiedlichen Standorten des Trinkwasser- und/oder Grundwassersystems entnommen. Vorzugsweise werden an mehr als 5 Standorten, bevorzugt mehr als 10 Standorten, Einzelindividuen des Tracerorganismus eingesammelt.

Anhand der polymorphen Nukleinsäureprimer kann dann eine Mikrosatellitenanalyse über eine Polymerasen-Kettenreaktion (PCR) erfolgen. Zur Identifizierung sind die Primer vorzugsweise mit einer Sonde gekoppelt, beispielsweise mit unterschiedlichen Farbstoffen wie CY7, IRD700 oder CY5. Vorzugsweise wird die Mikrosatellitenanalyse mit Probenmaterial des wenigstens einen Tracerorganismus durchgeführt, das aus dem Leitungsnetz und/oder dem Freiland des Trinkwasser- und/oder Grundwassersystems gewonnen wurde.

In einem weiteren Verfahrensschritt wird die Herkunft und/oder der Eintrags- oder Austragspfad des wenigstens einen Tracerorganismus in dem Trinkwasser- und/oder Grundwassersystem anhand der in der Mikrosatellitenanalyse gewonnenen Daten bestimmt. Dies erfolgt anhand des Vergleiches des Verwandtschaftsgrades der untersuchten Einzelindividuen bzw. Populationen. Je häufiger Paarungen von Individuen unterschiedlicher Populationen erfolgen, desto enger ist der Verwandtschaftsgrad. Tiere im Netz können so ihrer jeweiligen Ursprungspopulation zugeordnet werden. Anhand der Ermittlung der Verwandtschaftsgrade können nicht nur die Ausbreitungspfade im System ermittelt werden, sondern auch mit welcher Wahrscheinlichkeit die Tiere bestimmter Netzbereiche von mehreren Freilandpopulationen oder Populationen in einem Leitungsnetz abstammen. So können Populationen über Oberflächengewässer in das Grundwasser gelangen, wenn beispielsweise der Eintrag dieser Organismen über einen Brunnen erfolgt. Bei den Leitungsnetzen kann wiederum eine weitere Population aus einem Oberflächengewässer, beispielsweise bedingt durch Reparaturarbeiten an einem Leitungsnetz, in das Trinkwasserversorgungssystem eingetragen werden. Dort verursachen sie die anfangs beschriebenen Probleme. Anhand des erfindungsgemäßen Verfahrens ist eine exakte und objektive Aussage über die Herkunft und die Verbreitung in einem Trinkwasser- und/oder Grundwassersystem möglich und liefert damit die Grundlage für effiziente, zielgerichtete und kostenoptimierte Gegenmaßnahmen. Geeignete Probenentnahmestellen im Freiland sind beispielsweise Seen, Flüsse, Quellen oder Grundwassermessstellen.

Ein bevorzugter Tracerorganismus, der im vorliegenden Verfahren zur Anwendung kommt, ist die Wasserassel *Asellus aquaticus.* Die Mikrosatelliten wurden mittels Asselproben aus 15 verschiedenen Standorten in Deutschland, Österreich, der Schweiz und Großbritannien entwickelt. Bislang waren nur Mikrosatelliten höhlenbewohnender, taxonomisch unsicherer, südslowenischer Wasserasselpopulationen bekannt. Diese ließen sich für die mittel+europäischen Wasserasseln kaum oder gar nicht nutzen, da man häufig Kreuzamplifikationen feststellte. Aus diesem Grund basiert die Erfindung auch darauf, geeignete polymorphe Primer sowie Mikrosatellitenbereiche zu identifizieren, mit denen eine populationsgenetische Untersuchung, insbesondere eine Feststellung des Verwandtschaftsgrades unterschiedlicher Einzelindividuen eines Tracerorganismus von unterschiedlichen Probeentnahmestellen möglich ist.

Prinzipiell ist jeder Tracerorganismus beim erfindungsgemäßen Verfahren einsetzbar, bei dem eine Feststellung des Verwandtschaftsgrades anhand einer Mikrosatellitenanalyse möglich ist. Letztendlich sollen die von einem Standort entnommenen Einzelindividuen einer bestimmten Population zugeordnet werden, um so die Ausbreitungswege eines Kontaminanten in einem Trinkwasserrohrnetz oder im Grundwasser einzugrenzen oder aufzudecken.

Vorzugsweise kommen Organsimen zur Anwendung, die zu den Gattungen *Asellus,*

*Testacea, Copepoda, Nematoda, Rotatoria, Cladocera, Oligochaeta, Hydracarina, Ostracoda, Tardigrada, Turbellaria, Gastrotricha, Ciliata, Mollusca, Rotifera, Nemertini, Cnidaria, Amphipoda, Isopoda, Bathynellacea, Copepoda, Ostracoda, Annelida, Acari, Plathelminthes* gehören. Die Erfindung umfasst sämtliche Arten, die zu den oben genannten Gattungen gehören und als Kleinlebewesen eine Kontamination in einem Trinkwasser- und/oder Grundwassersystem verursachen können. Besonders bevorzugt ist die Analyse der Wasserassel, *Asellus aquaticus* aber auch andere Zielorganismen wie *Paracyclops fimbriatus, Eucyclops serrulatus, Acanthocyclops sensitivus, Acanthocyclops rhenanus.* Daneben sind jedoch auch weiter häufig vorkommende Tracerorganismen besonders bevorzugt, wie beispielsweise Schalenamöben (*Testacea*) oder Ruderfußkrebse (*Copepoda*), Fadenwürmer (*Nematoda*), Rädertierchen (*Rotatoria*), Blattfußkrebse (*Cladocera*), Ringelwürmer (*Oligochaeta*). Etwas weniger häufig, aber ebenso interessant sind Muschelkrebse (*Ostracoda*), Bärtierchen (*Tardigrada*), Strudelwürmer (*Turbellaria*), Bauchhärlinge (*Gastrotricha*) und Wimpertierchen (*Ciliata*). Ähnlich den Wasserasseln können sich verschiedene Spezies im Trinkwasserverteilungssystem vermehren, andere werden dagegen nur passiv transportiert und sterben aufgrund fehlender Nahrungsgrundlagen ab. In Abhängigkeit von der Art und Verfügbarkeit geeigneter Nahrung ist die Wahrscheinlichkeit, mit der Kleintiere in Trinkwasserverteilungssystemen registriert werden, unterschiedlich groß.

Das Vorkommen der Kleintiere im Trinkwasser mit ihren verschiedenen Lebensweisen und Lebensräumen sagt viel über die Herkunft des Rohwassers und über die Wirksamkeit der Wasseraufbereitung aus. Das Vorkommen von Wasserasseln in Trinkwasserverteilungssystemen ist in der Regel inhomogen, d.h. es gibt Rohrnetzabschnitte mit einer hohen Anzahl von Asseln und andere benachbarte Bereiche, in denen deutlich weniger Tiere feststellbar sind. Vermutlich sind hierfür unterschiedliche hydraulische Bedingungen oder das Nährstoffangebot ursächlich.

Anhand des erfindungsgemäßen Verfahrens ist es möglich, die Eintragswege solcher Kleinlebewesen, beispielsweise in das Trinkwassernetz, zu identifizieren. Solche Eintragswege basieren häufig auf einer Rückspülung von Schnellsandfiltern, Rohrnetzarbeiten oder Rohrleckagen.

Insofern trägt das erfindungsgemäße Verfahren dazu bei, gezielt Einträge von Kleinlebewesen in ein Trinkwasser- oder Grundwassersystem festzustellen und eine hydraulische Rohrnetzanalyse zu erleichtern. Insbesondere trägt das erfindungsgemäße Verfahren dazu bei, die Probleme der Sekundärverkeimung in Trinkwasserversorgungssystemen zu vermindern.

Die genetische Aufarbeitung der Proben erfolgt nach gängigen Methoden. Die Ergebnisse der Mikrosatellitenanalyse werden einer multivariaten, statistischen Analyse unterzogen. Dies ermöglicht eine Zuordnung von Einzelindividuen und Genotypen. Anhand der Auswertung können einzelne Individuen einer bestimmten Population zugeordnet werden, wodurch Eintragspfade und Verbreitungswege nachverfolgt werden können. Daneben ist auch eine verteilungsunabhängige Auswertung möglich, die mit wenigen Einzelindividuen pro Standort auskommt und eine statistisch belastbare Analyse erlaubt.

So können nahezu alle Individuen anhand der Mikrosatellitenanalyse individuell differenziert und zugeordnet werden. Dies belegt, dass das Verfahren sehr zuverlässig und genau funktioniert.

Die Qualitätssicherung des Trink- und/oder Grundwassersystems erfolgt erfindungsgemäß durch folgende Maßnahmen:
a. Ermittlung der Herkunft des wenigstens einen Tracerorganismus als Grundlage für dessen/deren Bekämpfung, und/oder
b. Nutzung des wenigstens einen Tracerorganismus als Tracer zur Ermittlung von Eintrags-und Ausbreitungspfaden potenzieller Kontaminanten in einem Trinkwasserversorgungssystem, und/oder
c. Nutzung des wenigstens einenTracerorganismus als Tracer bei der Festlegung von Wasserschutzgebieten, und/oder
d. Nutzung des wenigstens einen Tracerorganismus als Tracer zur Ermittlung hydraulischer Kurzschlüsse zwischen Oberflächengewässern und Trinkwassergewinnungsgebieten.

Daneben ist auch eine Ermittlung und Bewertung hydrologischer Interaktionen im Freiland möglich, was beispielsweise durch hydrogeologische Erkundungen z.B. im Rahmen einer Wasserschutzgebietsausweisung, einer Einzugsgebietseingrenzung oder durch eine Erfassung hydrologischer Veränderungen im Rahmen der Eingriffsregelung und der Umweltüberwachung (z.B. Feuchtgebietsmanagement) möglich ist. Somit ermöglicht das erfindungsgemäße Verfahren eine Erfassung und/oder Analyse von hydrologischen Veränderungen oder hydrologischen Wechselwirkungen im Leitungsnetz des Trinkwassersystems und/oder im Freiland des Grundwassersystems.

Wie bereits erwähnt, werden Einzelindividuen vorzugsweise von mehreren unterschiedlichen Standorten des Trinkwasser- und/oder Grundwassersystems entnommen. Vorzugsweise werden unterschiedliche Tracerorganismen von unterschiedlichen Probenstandorten des Trink- und/oder Grundwassersystems anhand ihrer Mikrosatelliten individuell differenziert, wobei anhand der vorkommenden Populationen der Tracerorganismen eine phylogenetische Landkarte der Tracerorganismen in dem Trinkwasser- und/oder Grundwassersystem generierbar ist. Anhand der phylogenetischen Landkarte kann festgestellt werden, zu welcher Population ein analysiertes Kleinlebewesen gehört und welchem Standort dieses Lebewesen zugeordnet werden muss. Etwaige undichte Stellen wie z.B. Rohrleckagen, undichte Brunnen oder sonstige Eintragspfade in einem Trinkwasserleitungsnetz können so leicht eingegrenzt werden.

Die Erfindung umfasst ferner neue, in dem Verfahren einsetzbare Nukleinsäureprimer für die Durchführung einer Polymerasen-Kettenreaktion, welche für die anschließende Mikrosatellitenanalyse erforderlich ist. Die Primer umfassen eine Nukleinsäuresequenz mit einer für eine PCR-Reaktion geeigneten Länge, wobei die Nukleinsäuresequenz der Primer auf einer der Sequenzen SEQ ID NO. 1 bis 10 oder deren Komplementärsequenzen basiert. Vorzugsweise sind die polymorphen Nukleinsäureprimer mit unterschiedlichen, voneinander unterscheidbaren detektierbaren Sonden markiert, wobei die Primersequenzen gewählt werden, dass sie bei der Polymerasen-Kettenreaktion DNA-Fragmente aus der Mikrosatellitenregion des Tracerorganismus mit einer Länge zwischen 60 und 420 bp erzeugen und DNA-Fragmente liefern, welche von der Größe unterschiedlich sind. Vorzugsweise umfasst die vorliegende Erfindung Nukleinsäureprimer, die eine Mikrosatellitensequenz eines der oben genannten Tracerorganismen flankieren. Bevorzugte Primer, welche für die Analyse von *Asellus aquaticus* entwickelt und eingesetzt wurden, umfassen eine Nukleinsäuresequenz, die auf einer der Nukleinsäuresequenzen SEQ ID NO. 1 bis 10 von *Asellus aquaticus* oder Homologen davon in anderen Organismen basiert. Vorzugsweise sind die auf Grundlage dieser Mikrosatelliten-Loci gewählten Primer zwischen 15 Bp und 25 Bp lang. Die Erfindung umfasst auch kleinere DNA-Nukleinsäuresequenzen dieser Primersequenzen, sofern sie für eine PCR noch funktionieren. Daneben sind auch längere Versionen von Primern möglich, sofern sie für eine PCR-Reaktion geeignet sind. In einer Variante können die Primer auch zusätzlich modifiziert sein. Die Erfindung umfasst auch die Auswahl der Mikrosatelliten-Loci zur Generierung solcher Primer. Diese für eine Mikroanalyse geeigneten Loci sind in den SEQ ID NO. 1 bis 10 beispielhaft für den Zielorganismus *Asellus aquaticus* gezeigt. Dies ist insbesondere darum wichtig, weil Kreuzreaktivitäten bei der PCR im Rahmen der Mikrosatellitenanalyse vermieden werden sollen. Zudem ist eine möglichst genaue Zuordnung des Organismus erforderlich. Die Primer werden für den erfindungsgemäßen Ansatz speziell und artenspezifisch entwickelt. Dadurch lassen sich die ausgewählten Einzelindividuen als Tracer für abiotische Faktoren (beispielsweise hydrologische Wechselwirkungen) oder in Trinkwasserversorgungsanlagen gezielt nutzen.

Die Erfindung wird in den nachfolgenden Beispielen näher erläutert.

### Material und Methoden

### 1. Auswahl von Tracerorganismen

An repräsentativen Stellen einer Versuchsanlage bzw. in einem Untersuchungsgebiet (Freiland) wurden Faunaproben entnommen, welche zumindest einen zu untersuchenden Tracerorganismus enthielten. Die Probenentnahme erfolgte über Netze mit einer Maschenweite von 64 µm im Durchlaufverfahren (bis zu 24 Stunden oder vorzugsweise > 5 m³) in Grundwassermessstellen mit sog. Netzsammlern,(phreatischen Plantonnetzen, Maschenweite 64 µm) oder in Freilandgewässern mit handelsüblichen Keschern (Maschenweite 0,5 - 1 mm).

Zur Auswahl der Tracerorganismen wurden die Tiere taxonomisch morphologisch bestimmt und geeignete Tracerorganismen ausgewählt.

### 2. Genetische Charakterisierung der potentiellen Tracerorganismen

### 2.1 Überprüfung auf kryptische Spezies

Im Grundwasser ist der Anteil sogenannter kryptischer Arten sehr hoch. Deshalb ist zu prüfen, ob dies auch für die ausgewählte, potentielle Indikatorart gilt. Dazu wird bei Individuen aus ganz Mitteleuropa (oder darüber hinaus) die DNA mittels der HotSHOT DNA-Methode nach Montero-Pau et al. (2008) extrahiert. Die morphologisch bestimmten Tiere wurden hierbei zunächst für jeweils 1 Stunde in 500 µl ddH₂O gelegt, um den Alkohol aus den Proben zu entfernen. Im Anschluss wurden den Proben jeweils 50 µl Lyse-Puffer mit einem pH von 12 (25 mM NaOH, 0,2 mM Na₂EDTA (pH 8) und ddH₂O) hinzugefügt. Es erfolgte eine Inkubation der Proben bei einer Temperatur von 95° C bei 300 rpm für 30 Minuten in einem Thriller Thermo-Inkubationsmischer der Firma PEQLAB. Anschließend wurden die Proben 4 bis 5 Minuten auf Eis gestellt und abzentrifugiert. Es folgte dann die Zugabe von jeweils 50 µl Neutralisationslösung mit einem pH von 5 (40 mM Tris-HCl und ddH₂O). Danach wurden die Proben gevortext, wieder abzentrifugiert und bei einer Temperatur von -20° C über Nacht eingefroren. Die Fällung der Proben zur Gewinnung der DNA erfolgte mit Isopropanol. Hierfür wurden den Proben jeweils 300 µl eiskalter Isopropanol (100 %) hinzugefügt. Die Reaktionsgefäße wurden 5-6 Mal per Hand gedreht und dann bei 16000 g für 10 Minuten bei 20° C zentrifugiert. Der Überstand wurde jeweils mit einer Pipette abgenommen und die Proben im Heizblock bei 40° C getrocknet. Danach erfolgte die Zugabe von jeweils 30 µl sterilem ddH₂O. Die Proben wurden in den Thermo-Inkubationsmischer bei 450 rpm für 20 Minuten bei 55° C gestellt und danach bei 4° C im Kühlschrank gelagert.

Mit der so aus dem Zielorganismus extrahierten DNA wurden die PCR-Reaktionen durchgeführt. Hierzu wurde zuerst ein Mastermix angefertigt, welcher aus sterilem Wasser, Magnesiumchlorid, Puffer, dNTPs, Forward- und Reverse-Primern (COI sowie 12SrRNA) sowie der TAQ-Polymerase besteht. Der Mastermix wurde kurz gevortext. Anschließend wurden 18 µl des Mastermix mit 2 µl des Extraktes in PCR-Eppendorf-Tubes pipettiert und abzentrifugiert. Die Proben wurden in den Thermozykler gestellt und das folgende PCR-Programm gestartet: Programmschritt 1 (95° C für 2 min), Programmschritt 2 (Denaturierung: 95° C für 1 min), Programmschritt 3 (Primer-Annealing: 40° C für 1 min), Programmschritt 4 (Elongation: 72° C für 1,5 min), Programmschritt 5 (72° C für 7 min). Die Programmschritte zwei bis vier werden 35fach wiederholt.

Zur Kontrolle ob ausreichende Mengen PCR-Produkt amplifiziert worden sind, wurde eine Gelelektrophorese durchgeführt. Hierbei wurde ein 1,5%iges Gel aus 0,45 g Agarose (peq Gold universal Agarosepulver) und 30 ml 1xTBE (Herstellung 10xTBE: 54 g TrisBase, 27,5 g Borsäure und 20 ml 0,5 M EDTA auf 500 ml ddH2O; Herstellung 1xTBE: 50 ml 10xTBE mit 450 ml ddH2O) hergestellt. 1 µl Ladepuffer wurde mit 2 µl des PCR-Produktes auf einem Stück Parafilm vermischt und anschließend in die Geltaschen pipettiert. Außerdem wurde eine Tasche pro Gelreihe mit 1 µl eines Markers (100 bp DNA-Leiter) befüllt. Danach wurde die Gelkammer mit 0,5%igem TBE-Puffer aufgefüllt und für 30 Minuten eine Spannung von 100 V angelegt. Anschließend erfolgte eine zehnminütige Färbung des Gels in einem Ethidiumbromid-Bad (50 µl 10 mg/ml Ethidiumbromid; 500 ml 1xTBE). Nach einer kurzen Entfärbungsphase in einem Entfärbebad (1xTBE) wurde das Gel abschließend unter UV-Licht (Wellenlänge 302 nm) digital fotografiert.

Die ausgewählten PCR-Produkte wurden sequenziert. Mit Hilfe des Programms Geneious (Version 5.3.6) wurden die Sequenzdaten editiert und verglichen. Alle Consensus-Sequenzen wurden auf eine gemeinsame Länge (615 bp) gebracht und bildeten den Datensatz für die weiteren statistischen Auswertungen. Mit dem Programm DNA Sequence Polymorphism (DnaSP Version 5.10) wurde anhand des erstellten Datensatzes ein Roehl-Data-File (rdf-Datei) generiert, welcher wiederum in die Phylogenetic Network Software (Version 4.612) importiert wurde, um die Haplotypen zu bestimmen und um ein Haplotypen-Netzwerk (Median-Joining) zu kalkulieren. Der phylogenetische Baum wurde mittels Neighbour-Joining-Test nach dem Kiumara-2- Parameter-Modell und der Einstellung "pairwise deletion" sowie 1000-Bootstrap-Replikaten erstellt. Die phylogenetische Auswertung zeigte dann, ob man es mit kryptischen Arten zu tun hat oder nicht und ob regionale Muster erkennbar sind.

Von den 13 natürlich vorkommenden *Asellus aquaticus*-Populationen wurden in dem hier aufgeführten Beispiel insgesamt 172 Individuen in ganz Europa gesammelt und deren DNA-Sequenzen ermittelt und über die Softwareprogramme Geneius und Network verglichen. Die Analyse ergab, dass es sich bei den getesteten Populationen um eine gut durchmischte Population handelte. Kryptische Arten wurden nicht festgestellt.

### 2.2 Entwicklung von polymorphen Primern

Für die Mikrosatellitenanalyse wurden PCR-Primer entwickelt, welche auf Nukleinsäuresequenzen basieren, die von *Asellus aquaticus* stammen. Dieser Organsimus ist nur beispielhaft; das Verfahren funktioniert auch mit anderen Zielorganismen. Dazu wurden Einzelindividuen von insgesamt 13 Standorten entnommen. Für die Mikrosatellitenanalyse wurde die DNA aus dem Organismus extrahiert und sequenziert. Für die Primerentwicklung wurden die nachfolgend aufgeführten Nukleinsäuresequenzen (in 5'-3'-Richtung) herangezogen. Die für die PCR eingesetzten Primer-Sequenzen sind unterstrichen. Dabei handelt es sich bei der ersten unterstrichenen Sequenz um die Forward (5'-3')-Primer. Die Backward-Primer ergeben sich aus der jeweiligen Komplementärsequenz der zweiten unterstrichenen Sequenz.
SEQ ID NO.1
SEQ ID NO.2
SEQ ID NO.3
SEQ ID NO.4
SEQ ID NO.5
SEQ ID NO.6
SEQ ID NO.7
SEQ ID NO.8
SEQ ID NO.9
SEQ ID NO.10

### 2.3 Genetische Aufarbeitung der Proben

Um die DNA zu extrahieren, wurde das Verfahren der Plattenextraktion verwendet. Dieses Verfahren wird bei der Aufarbeitung großer Probenmengen genutzt, da hier knapp 200 Proben gemeinsam aufgearbeitet werden können. Zunächst wurden 50µl Probenpuffer in die Vertiefungen (Wells) einer Probenplatte pipettiert. Dann gab man das Tiermaterial (2 Beine pro Tier) mit der Federstahlpinzette in den Puffer und inkubiert die Platten ÜN bei 56°C, lid at 80°C. Die beiden Platten wurden nun mittels Plattenzentrifuge 1 min, 1000 rpm zentrifugiert. Im Anschluss wurde 100µl Bindepuffer mit der Multichannel-Pipette aufgeben und zum Mischen 3mal auf- und abpipettiert. Es folgte ein Zentrifugieren in der Plattenzentrifuge für 20 Sek bei 1000 rpm. Mit der Multichannel-Pipette wurden nach der Zentrifugation 130 µl aus jedem Well entnommen, in GF-Platten überführt und mit selbstklebender Folie verschlossen. Es schloss sich ein Zentrifugationsschritt in der Plattenzentrifuge für 5 min bei 5000 rpm an. Zum Waschen wurden 180µl Puffer hinzupipettiert, die Platten wieder mit selbstklebender Folie verschlossen und erneut in der Plattenzentrifuge 2 Min bei 5000 rpm zentrifugiert. Mit der Multistep-Pipette wurden 750µl WB-Puffer zugegeben, die Platten mit selbstklebender Folie verschlossen und in der Plattenzentrifuge 5 Min bei 5000 rpm zentrifugiert. Im Anschluss wurde die selbstklebende Folie abgezogen, um das Vakuum zu entfernen. Die Platten wurden erneut in der Plattenzentrifuge für 5 Min bei 5000 rpm zentrifugiert. Danach wurden die Platten 30 Min bei 56°C inkubiert. Nach der Inkubation wurden die GF-Platten auf neue Platten aufgesetzt und mit der Multistep-Pipette 50 µl 56°C-warmes A.dest. aufpipettiert und eine Minute bei Raumtemperatur inkubiert. Die Elution erfolgte mittels Zentrifugation für 5 Min bei 5000 rpm. Für die Fragmentanalyse wurde ein Mastermix bestehend aus dem 400er Größenstandard (0,5µl pro Probe) und SLS-Puffer (29,5µl pro Probe) angesetzt und eine Probenplatte mit jeweils 29,5µl pro Vertiefung Mastermix belegt. Mit der Multichannel-Pipette wurde in jedes Well 0,5µl DNA pipettiert, ein Tropfen Mineralöl auf jede Probe gegeben, die Platte kurz zentrifugiert und dann auf den CEQ-Sequenzierer geladen. Die Daten wurden mit der CEQ-Software ausgewertet.

Bei den 182 dem Modellversorgungssystem entnommenen Tieren wurde dann eine DNA-Extraktion mit nachfolgender Fragmentanalyse (unter Verwendung der neu designten polymorphen Primer) unternommen. Die so gewonnen Daten wurden dann mit Hilfe des Programms Geneious (Version 5.3.6) optimiert.

### 2.4 Multivariate, statistische Analyse der Daten

Die Daten wurden mittels eines GenAlEx (Genetic Analysis in Excel) ausgewertet. GenAlEx ist eine Freeware-Software speziell für populationsgenetische Analysen, die in Microsoft Excel ausgeführt wird. Sie erlaubt die Zuordnung von Einzelindividuen und Genotypen. Dadurch kann ermittelt werden, welcher Population ein Einzelindividuum zuzuordnen ist.

Parallel dazu erfolgte eine Auswertung mit der ökologischen Software PRIMER, die sehr robuste, verteilungsunabhängige Ergebnisse liefert. Der Vorteil dieses zusätzlichen Verfahrens liegt darin, dass auch mit wenigen Tieren je Standort eine statistisch belastbare Analyse durchgeführt und die genetischen Daten dabei mit beliebig vielen Umweltparametern verknüpft werden können.

Die Auswertung der Daten mittels GenAlEx (Genetic Analysis in Excel) ergab, dass von den 182 bearbeiteten Tieren 180, also 99 % der Individuen, anhand der Mikrosatelliten individuell differenzierbar sind. Dieses Ergebnis belegt, dass das Verfahren zuverlässig und sehr genau funktioniert. Die weiteren Auswertungen mit GenAlEx und PRIMER zu den Verteilungs- und Ausbreitungsmustern der Tiere verliefen ebenfalls erfolgreich.

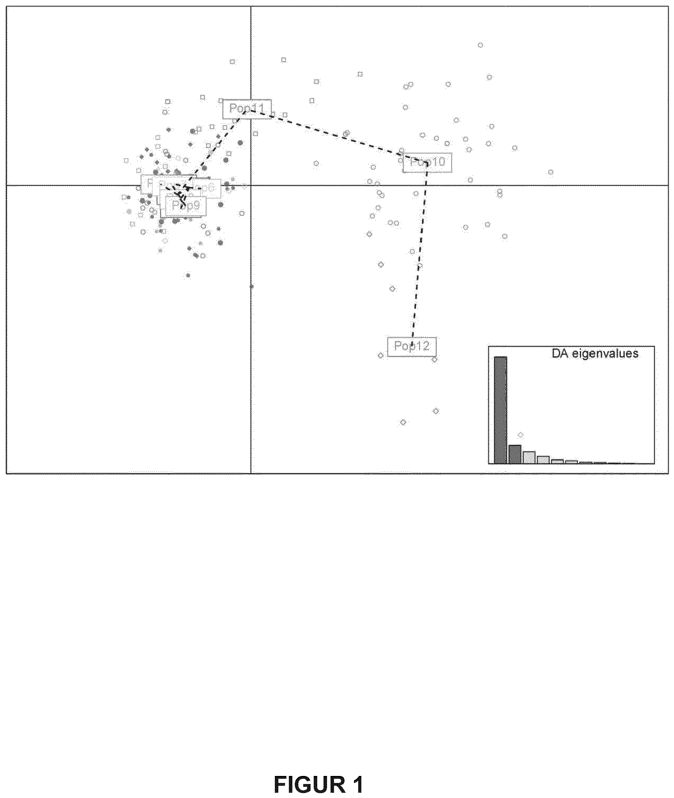

In Fig. 1 ist das Ergebnis einer Mikrosatellitenanalyse gezeigt, anhand derer der Verwandtschaftsgrad der untersuchten Einzelindividuen und eine Zuordnung zu unterschiedlichen Populationen möglich ist. Gezeigt sind zwölf unterschiedliche Populationen des Zielorganismus *Asellus aquaticus.* Die Populationen 1 bis 9 sind Populationen aus dem Leitungsnetz, die Populationen 10 bis 12 stammen aus dem Freiwasser. Gezeigt ist das Verteilungsmuster der zwölf untersuchten Populationen. Jedes Symbol steht für ein Individuum. Tiere aus derselben Population bzw. demselben Standort sind in gleicher Färbung wiedergegeben. Die Abstände zwischen den Individuen spiegeln ihre genetische Distanz wider.

Die drei Freiwasserproben (Populationen 10 bis 12) unterscheiden sich bezüglich ihrer genetischen Charakterisierung nicht nur gegenüber den Proben des Leitungsnetzes, sondern auch untereinander deutlich. So ist in der Fig. 1 ein Ost-West-Gradient zu erkennen. Weiterhin sind die Netzpopulationen stark durch die Freilandpopulation 10 geprägt.

Idealerweise werden die Zielorganismen möglichst umfassend gesammelt (z.B. deutschland-oder europaweit) und morphologisch einwandfrei bestimmt. Bei der Ermittlung des Artstatus muss abgeklärt werden, ob es sich um eine einzelne Art oder einen Artenkomplex handelt. Dies ist insbesondere für die nachfolgende genetische Analyse notwendig, da diese in der Regel immer nur eine bestimmte Art detektiert. Dies ist jedoch abhängig von der genetischen Verwandtschaft und den eingesetzten Primern.

Je nach Größe des Zielorganismus kann es auch notwendig sein, die DNA-Extraktionsbedingungen zu modifizieren, um die relativ geringen DNA-Mengen zu extrahieren. Eingesetzt wurde das DNA-Extraktionsverfahren gemäß dem Hotshot-Verfahren nach Montero-Pau (2008).

## Patentansprüche

1. Verfahren zur Qualitätssicherung bei der Trinkwasser- und/oder Grundwasserversorgung in Trinkwasser- und/oder Grundwassersystemen durch eine populationsgenetische Analyse von Mikrosatelliten in der DNA von wenigstens einem Tracerorganismus, umfassend die Schritte:
a. Auswählen wenigstens eines für das jeweilige Trinkwasser- und/oder Grundwassersystem geeigneten Tracerorganismus durch eine taxonomische Untersuchung und/oder genetische Charakterisierung,
b. Bereitstellen von polymorphen Nukleinsäure-Primern für die auf einer Polymerasen-Kettenreaktion (PCR) basierenden Analyse von Mikrosatelliten des wenigstens einen Tracerorganismus,
c. Durchführen einer Mikrosatellitenanalyse mit Probenmaterial des wenigstens einen Tracerorganismus, das aus dem Leitungsnetz und/oder dem Freiland des Trinkwasser- und/oder Grundwassersystems gewonnen wurde,
d. Bestimmen der Herkunft und/oder der Eintragungspfade und/oder der Ausbreitungspfade des wenigstens einen Tracerorganismus in dem Trinkwasser- und/oder Grundwassersystem anhand der in der Mikrosatellitenanalyse gewonnenen Daten.

2. Verfahren nach Anspruch 1, **dadurch gekennzeichnet, dass** der wenigstens eine Tracerorganismus ausgewählt ist aus der Gruppe bestehend aus *Asellus, Testacea, Copepoda, Nematoda, Rotatoria, Cladocera, Oligochaeta, Hydracarina, Ostracoda, Tardigrada, Turbellaria, Gastrotricha, Ciliata, Mollusca, Rotifera, Nemertini, Cnidaria, Amphipoda, Isopoda, Bathynellacea, Copepoda, Ostracoda, Annelida, Acari, Plathelminthes.*

3. Verfahren nach Anspruch 1 oder 2, **dadurch gekennzeichnet, dass** der wenigstens eine für das jeweilige Trinkwasser- und/oder Grundwassersystem geeignete Tracerorganismus durch eine phylogenetische Merkmalsanalyse ausgewählt wird, um festzustellen, ob eine kryptische Art vorliegt oder nicht, wobei beim Vorliegen einer kryptischen Art der Tracerorganismus weiter daraufhin untersucht wird, ob regionale Verbreitungsmuster erkennbar sind oder nicht, wobei der Tracerorganismus dann für die weitere Mikrosatellitenanalyse herangezogen wird, wenn keine kryptische Art vorliegt oder eine kryptische Art ein regionales Verbreitungsmuster aufweist.

4. Verfahren nach einem der Ansprüche 1 bis 3, **dadurch gekennzeichnet, dass** die über die Mikrosatellitenanalyse gewonnenen Daten einer multivariaten Auswertung unterzogen werden, wobei eine Zuordnung der untersuchten Einzelindividuen jedes Tracerorganismus zu einer Population erfolgt, wobei anhand der Verteilung der Populationen der Tracerorganismen die Herkunft und/oder der Eintragungspfad und/oder der Ausbreitungspfad des wenigstens einen Tracerorganismus in dem Trinkwasser- und/oder Grundwassersystem ermittelt wird.

5. Verfahren nach einem der Ansprüche 1 bis 4, **dadurch gekennzeichnet, dass** die Qualitätssicherung des Trink- und/oder Grundwassersystems erfolgt durch:
a. Ermittlung der Herkunft des wenigstens einen Tracerorganismus als Grundlage für dessen/deren Bekämpfung, und/oder
b. Nutzung des wenigstens einen Tracerorganismus als Tracer zur Ermittlung von Eintrags- und Ausbreitungspfaden potenzieller Kontaminanten in einem Trinkwasserversorgungssystem, und/oder
c. Nutzung des wenigstens einen Tracerorganismus als Tracer bei der Festlegung von Wasserschutzgebieten, und/oder
d. Nutzung des wenigstens einen Tracerorganismus als Tracer zur Ermittlung hydraulischer Kurzschlüsse zwischen Oberflächengewässern und Trinkwassergewinnungsgebieten.

6. Verfahren nach einem der Ansprüche 1 bis 5, **dadurch gekennzeichnet, dass** eine Erfassung und/oder Analyse von hydrologischen Veränderungen oder hydrologischen Wechselwirkungen im Leitungsnetz des Trinkwassersystems und/oder im Freiland des Grundwassersystems erfolgt.

7. Verfahren nach einem der Ansprüche 1 bis 6, **dadurch gekennzeichnet, dass** zur Gewinnung des Probenmaterials Einzelindividuen des Tracerorganismus aus mehreren unterschiedlichen Standorten des Trinkwasser- und/oder Grundwassersystems entnommen werden.

8. Verfahren nach einem der Ansprüche 1 bis 7, **dadurch gekennzeichnet, dass** unterschiedliche Tracerorganismen von unterschiedlichen Probenstandorten des Trink- und/oder Grundwassersystems anhand ihrer Mikrosatelliten individuell differenziert werden, wobei anhand der Vorkommen der Populationen der Tracerorganismen eine phylogenetische Landkarte der Tracerorganismen in dem Trinkwasser- und/oder Grundwassersystem generiert wird.

9. Verfahren nach einem der Ansprüche 1 bis 8, **dadurch gekennzeichnet, dass** die polymorphen Nukleinsäureprimer mit unterschiedlichen, voneinander unterscheidbaren detektierbaren Sonden markiert sind, wobei die Primersequenzen so gewählt werden, dass sie bei der Polymerasen-Kettenreaktion DNA-Fragmente aus der Mikrosatellitenregion des Tracerorganismus mit einer Länge zwischen 60 und 420 bp erzeugen und DNA-Fragmente liefern, welche von der Größe unterschiedlich sind.

10. Verwendung von Nukleinsäure-Primern, umfassend eine Nukleinsäuresequenz mit einer für eine PCR-Reaktion geeigneten Länge, wobei die Nukleinsäuresequenz der Primer eine der Sequenzen SEQ ID NO. 1 bis 10 oder deren Komplementärsequenzen umfasst, für die Durchführung einer Polymerasen-Ketten-Reaktion (PCR) vermittelten Mikrosatellitenanalyse zur Analyse und/oder Charakterisierung von Tracerorganismen in einem Trinkwasser- und/oder Grundwassersystem.

11. Verwendung nach Anspruch 10, **dadurch gekennzeichnet, dass** der Primer mit einer Farbstoffsonde kovalent gekoppelt ist.

## Claims

1. A method for quality assurance of drinking water and/or groundwater supplies in drinking water and/or groundwater systems by a population genetic analysis of microsatellites in the DNA of at least one tracer organism, comprising the following steps:
a. selecting at least one tracer organism suitable for the respective drinking water and/or groundwater system by a taxonomic analysis and/or genetic characterisation,
b. providing polymorphic nucleicacid primers for an analysis based on a polymerase chain reaction (PCR) of microsatellites of the at least one tracer organism,
c. carrying out a microsatellite analysis with sample material of the at least one tracer organism, which has been obtained from the supply network and/or the open land of drinking water and/or groundwater system,
d. determining the origin and/or the entry paths and/or the propagation paths of the at least one tracer organism in the drinking water and/or groundwater system based on the data obtained from the microsatellite analysis.

2. The method according to claim 1, **characterised in that** the at least one tracer organism is selected from the group consisting of *Asellus, Testacea, Copepoda, Nematoda, Rotatoria, Cladocera, Oligochaeta, Hydracarina, Ostracoda, Tardigrada, Turbellaria, Gastrotricha, Ciliata, Mollusca, Rotifera, Nemertini, Cnidaria, Amphipoda, Isopoda, Bathynellacea, Copepoda, Ostracoda, Annelida, Acari, Plathelminthes.*

3. The method according to claim 1 or 2, **characterised in that** the at least one tracer organism suitable for the respective drinking water and/or groundwater system is selected through a phylogenetic feature analysis for the purpose of determining whether a cryptic nature exists or not, whereby in the presence of a cryptic tracer organism it is further examined whether regional distribution patterns can be recognized or not, the tracer organism is then used for further microsatellite analysis, if there is no cryptic nature or a cryptic type has a regional distribution pattern.

4. The method according to claims 1 to 3, **characterised in that** the data obtained via microsatellite analysis are subjected to a multivariate analysis, wherein a mapping of the tested individual compounds of each tracer organism is carried out in a population, wherein based on the distribution of the populations of tracer organisms, the origin and/or the entry path and/or the propagation path of the at least one tracer organism are determined in the drinking water and/or groundwater system.

5. The method according to claims 1 to 4, **characterised in that** the quality control of the drinking water and/or groundwater system is carried out by:
a. determining the origin of the at least one tracer organism as a basis for its / their control, and/or
b. utilizing the at least one tracer organism as a tracer for determining the entry and propagation paths of potential contaminants in a drinking water supply system, and/or
c. utilizing the at least one tracer organism as a tracer while determining the water protection areas, and/or
d. utilizing the at least one tracer organism as a tracer for determining of hydraulic short circuits between surface waters and drinking water generation areas.

6. The method according to one of claims 1 to 5, **characterised in that** a detection and/or analysis of hydrological changes or hydrological interactions is carried out in the distribution network of the drinking water system and/or in open land of the groundwater system.

7. The method according to any one of claims 1 to 6, **characterised in that** for recovering the sample material individual compounds of the tracer organism are taken from several different locations of the drinking water and/or groundwater system.

8. The method according to one of claims 1 to 7, **characterised in that** different tracers organisms of different sample locations of drinking water and/or groundwater system are differentiated individually with the help of their microsatellites, whereby based on the occurrence of populations of tracer organisms, a phylogenetic map of the tracer organisms in drinking water and/or the groundwater system is generated.

9. The method according one of claims 1 to 8, **characterised in that** the polymorphic nucleic acid primer are labelled with different, mutually distinguishable and detectable probes, wherein the primer sequences are so selected that during the polymerase chain reaction, they generate DNA fragments from the microsatellite region of the tracer organism with a length between 60 and 420 bp and deliver DNA fragments, which are different in size.

10. Use of nucleic acid primers comprising a nucleic acid sequence with a length suitable for a PCR reaction, whereby the nucleic acid sequence of the primers comprises one of the sequences SEQ ID NO. 1 to 10 or their complementary sequences for performing a microsatellite analysis mediated by a polymerase chain reaction (PCR) for analysis and/or characterizing tracer organisms in a drinking water and/or the groundwater system.

11. The use according to claim 10, **characterised in that** the primer is covalently coupled with a dye probe.

## Revendications

1. Procédé de contrôle de la qualité lors de l'approvisionnement en eau potable et/oû en eau souterraine par le biais d'une analyse de génétique des populations de microsatellites dans l'ADN d'au moins un organisme traceur, comprenant les étapes consistant à :
a. sélectionner au moins un organisme traceur approprié pour le système respectif d'eau potable et/ou d'eau souterraine par le biais d'une étude taxonomique et/ou d'une caractérisation génétique,
b. préparer des amorces d'acide nucléique polymorphes pour l'analyse basée sur une réaction en chaîne par polymérases (PCR) de microsatellites du au moins un organisme traceur,
c. réaliser une analyse de microsatellites avec un matériau échantillon du au moins un organisme traceur, lequel est obtenu à partir du réseau de distribution et/ou de l'environnement du système d'eau potable et/ou d'eau souterraine,
d. déterminer l'origine et/ou la voie d'introduction et/ou la voie de dissémination du au moins un organisme traceur dans le système d'eau potable et/ou d'eau souterraine sur la base des données obtenues lors de l'analyse de microsatellites.

2. Procédé selon la revendication 1, **caractérisé en ce que** le au moins un organisme traceur est sélectionné parmi le groupe constitué d'*Asellus,* de *Testacea,* de *Copepoda,* de *Nematoda,* de *Rotatoria,* de *Cladocera, d'Oligochaeta, d'Hydracarina, d'Ostracoda,* de *Tardigrada,* de *Turbellaria,* de *Gastrotricha,* de *Ciliata,* de *Mollusca,* de *Rotifera,* de *Nemertini,* de *Cnidaria*, d'*Amphipoda*, d'*Isopoda,* de *Bathynellacea,* de *Copepoda*, d'*Ostracoda*, d'*Annelida*, d'*Acari,* de *Plathelminthes.*

3. Procédé selon la revendication 1 ou 2, **caractérisé en ce que** le au moins un organisme traceur approprié pour le système respectif d'eau potable et/ou d'eau souterraine est sélectionné par le biais d'une analyse des caractéristiques phylogénétiques, pour établir si une espèce cryptique est présente ou non, en présence d'une espèce cryptique, l'organisme traceur étant ensuite davantage étudié, si des modèles de propagation régionaux peuvent être identifiés ou non, l'organisme traceur étant ensuite utilisé pour l'analyse ultérieure de microsatellites, lorsque aucune espèce cryptique n'est présente ou qu'une espèce cryptique présente un modèle de propagation régional.

4. Procédé selon l'une des revendications 1 à 3, **caractérisé en ce que** les données obtenues par le biais de l'analyse de microsatellites font l'objet d'un dépouillement à variables multiples, où une attribution des individus particuliers étudiés de chaque organisme traceur à une population est effectué, où sur la base d'une répartition des populations des organisme traceurs, l'origine et/ou la voie d'introduction et/ou la voie de dissémination du au moins un organisme traceur dans le système d'eau potable et/ou d'eau souterraine est(sont) déterminée(s).

5. Procédé selon l'une des revendications 1 à 4, **caractérisé en ce que** le contrôle de la qualité du système d'eau potable/d'eau souterraine est effectué par le biais :
a. d'une détermination de l'origine du au moins un organisme traceur comme base pour pouvoir la/le combattre, et/ou
b. d'une utilisation du au moins un organisme traceur en tant que traceur pour déterminer les voies d'introduction et de dissémination d'éventuels contaminants dans un système d'approvisionnement en eau potable, et/ou
c. d'une utilisation du au moins un organisme traceur en tant que traceur dans l'établissement de zones de protection des eaux, et/ou
d. d'une utilisation du au moins un organisme traceur en tant que traceur pour déterminer des courts-circuits hydrauliques entre eaux de surface et zones d'exploitation d'eau potable.

6. Procédé selon l'une des revendications 1 à 5, **caractérisé en ce qu'**un recensement et/ou une analyse de changements hydrologiques ou d'interactions hydrologiques dans le réseau de distribution du système d'eau potable et/ou dans l'environnement du système d'eau souterraine est(sont) effectué(s).

7. Procédé selon l'une des revendications 1 à 6, **caractérisé en ce que**, pour l'obtention du matériau échantillon, des individus particuliers de l'organisme traceur sont prélevés sur plusieurs sites différents du système d'eau potable et/ou d'eau souterraine.

8. Procédé selon l'une des revendications 1 à 7, **caractérisé en ce que** différents organismes traceurs provenant de différents sites d'échantillonnage du système d'eau potable et/ou d'eau souterraine sont individuellement différenciés sur la base de leurs microsatellites, où en fonction de la présence des populations des organismes traceurs, une carte géographique phylogénétique des organismes traceurs dans le système d'eau potable et/ou d'eau souterraine est générée.

9. Procédé selon l'une des revendications 1 à 8, **caractérisé en ce que** les amorces d'acide nucléique polymorphes sont marquées avec différentes sondes détectables et différentiables les unes des autres, les séquences d'amorces étant sélectionnées de telle sorte que, pendant la réaction en chaîne par polymérases, elles produisent des fragments d'ADN dans la région des microsatellites de l'organisme traceur ayant une longueur comprise entre 60 et 420 pb et fournissent des fragments d'ADN, qui diffèrent en taille.

10. Utilisation d'amorces d'acide nucléique, comprenant une séquence d'acides nucléiques ayant une longueur appropriée pour une réaction PCR, la séquence d'acides nucléiques de l'amorce comprenant l'une des séquences à numéros d'identification SEQ ID 1 à 10 ou leurs séquences complémentaires, pour la réalisation d'une analyse de microsatellites basée sur une réaction en chaîne par polymérases (PCR) pour analyse et/ou caractérisation d'organismes traceurs dans un système d'eau potable et/ou d'eau souterraine.

11. Utilisation selon la revendication 10, **caractérisée en ce que** l'amorce est liée de manière covalente à une sonde de colorant.
